# EUROPEAN PATENT APPLICATION

(11) **EP 1 160 310 A1**
(43) Date of publication of application: **05.12.2001**
(21) Application number: 01304605.7
(22) Date of filing: 24.05.2001
(51) Int. Cl.: C11D 3/382, A61K 7/48

(54) **Detergent composition**

(30) Priority: 29.05.2000 JP 2000158272
(71) Applicant: Teizo, Satoh, Shijonawate-shi, Osakafu (JP)
(72) Inventor: Teizo, Satoh, Shijonawate-shi, Osakafu (JP)
(74) Representative: Bankes, Stephen Charles Digby

(57) **Abstract**

A detergent composition, which may be solid or liquid detergent, comprises a dispersion ash-like powders of hulls and/or carbonised or pyrolysed powders of such hulls, especially rice hulls respectively or in combination, in one or more surfactants or in water. The powders can be dispersed evenly on the surface of the material to be cleaned, without harming surfaces such as human skin, providing for even absorption and removal of oils and fats while enabling even and effective cleaning.

## Description

The present invention relates to a detergent which provides for easier removal of dirt and other contaminants such as oils and fats when washing hair, human skin or goods.

Detergents for various purposes generally contain a surface active agent, which agent is effective for easier removal by separation of dirty spots like oils and fats from the surfaces of the human skin or solid materials such crockery, work surfaces or textiles. As surface active agents there are, for instance, anionic surfactants, cationic surfactants, non-ionic surfactants, amphoteric surfactants and so on.

Detergent compositions typically also contain an absorbing material, which is porous and assists in separation and recovery of the oils from the mixture of oil and water. For this purpose, in addition to the known synthetic resins, it is known to use dried hulls , notably rice hulls.

Rice hulls comprise both embryo and albumen from the ears of rice plants and are known as end parts of rice plants. They are by-products made in large quantity by threshing after the collection of rice. Hulls, which have been dried, have rough surfaces with a dimension which is large and a specific gravity which is small so that they have the characteristics of getting selectively absorbed against an oil layer floating on a water surface, making them suitable for oil and fat. (One example is Japanese Patent No. 2706170).

In addition, there is disclosed in the public gazette of Japanese Patent Publication No. Heisei 3 (1991)-77239, the use of the micro powders of pulverised hulls and diatomaceous earth of approximately 0.1 ∼ 100 *µ*m particle size, combined to be used as a washing agent for family use together with the surface active agent.

Furthermore, use in cosmetic soap of absorbing agents such as micro powders is known.

However, among the conventional detergents above described, those which have included pulverised hulls with the surface active agent usually contain hulls of variable particle diameter or hulls which are unevenly dispersed. Consequently, the complete absorption and removal of contaminants such as fat and oil has been difficult to attain.

In addition, a cosmetic soap combined with micro powders of charcoal tends not to feel natural on the skin surface because of the low hydrophilicity of the powders, thus making the surface feel rough or lumpy when washing the hands with such a soap.

In addition, when applying such a detergent onto the surface of the windshield of automobiles as a removing agent for oil films, there is a problem that uneven effects of cleaning appear on the glass surface on which the detergent is dispersed unevenly, causing smearing.

An object of the present invention is therefore to provide a detergent capable of removing dirt effectively by resolving the above-mentioned problems, wherein the detergent is evenly absorbed into the dirty spots such as oils and fats attached onto the surface of the human skin or items while washing them.

It is a further object of the present invention to provide a detergent capable of obtaining cleaning effects evenly and sufficiently, wherein the detergent is definitely absorbed onto the oils and fats attached onto the surface of the human skin or glass and other items without damaging the surface.

The present invention concerns a detergent in which one or more surfactants are combined with ash-like powders of hulls or carbide powders of hulls, or both, dispersed into the surfactants.

The above-mentioned detergent consists of ash-like powders of the hulls or carbonised or pyrolysed powders of the hulls, or a combination of two, which are softer than ash-like powders of wood or charcoal powders that have been conventionally used, thus comprising micro powders of the same diameter of the particles while having hydrophilicity so that they get dispersed evenly on the surface of the materials to be cleaned without even harming the surface of soft materials to be cleaned, such as human skin, while providing for the thorough and even absorption and removal of oils and fats.

In addition, in order to have the above-mentioned operation in a definite manner, there is provided a detergent combining the ash-like powders of the hulls or the carbide powders of the hulls or using the compound powders of 3 ∼ 85 volume percent based on both of them with the solid detergent in a dispersed state.

In addition, when making a liquid detergent comprising the above said surfactants, there is provided a detergent combining the ash-like powders of the hulls or carbide powders of the hulls or using the compound powders of 3 ∼ 85 volume percent based on both of them with the liquid detergent in a dispersed state.

Furthermore, when adopting water instead of the above said surfactant, there can be provided a detergent combining the ash-like powders of the hulls or the carbide powders of the hulls or using the compound powders of 5 ∼ 85 volume percent based on both of them with water in a dispersed state. According to the detergent pertaining to the present invention, wherein a given amount of the ash-like powders of the chaffs or the carbide powders of the hulls or the compound powders using both of them are combined with the solid or liquid detergent or surfactants, powders are lubricated by the detergent or the surfactants, so that the surface of the human skin or the articles will not be harmed even when washing them with hands using the detergent thereon and oils and fats can be cleaned up with water effectively.

### [Preferred Embodiment of the Invention]

One example of the preferred embodiment of the present invention will be below explained.

The surfactant to be used for the detergent of the present invention may preferably be chosen from well-known ionic surfactants, nonionic surfactants, amphoteric surfactants, whose kinds are not limited, however, when these are used as a skin detergent of the liquid (including the fluid liquid material like cream or emulsion), the surfactants, so excellent in stability as to keep evenly dispersed without being separated, even though they have been left untouched for a long time, may preferably be used, thus for example, nonionic surfactants are preferable.

For example, a nonionic surfactant may be the one using fatty acid or higher alcohol for hydrophilicity or based on polyhydric alcohol or high-molecular compound as hydrophilic groups while it is a surface active agent combined for conventional cosmetic products.

The solid detergent or liquid detergent to be used for the present invention concerns a detergent already prepared according to the use of the detergent or items to be cleaned or a single material capable of being used as a detergent for washing with water and may or may not include a surfactant. Furthermore, they can adopt the liquid detergent comprising a single component of glycerin.

For instance, the process of the solid soap illustrating one example of the solid detergent is shown as below. After the oils and fats comprising the suet and coconut oil are reacted with the sodium hydroxide, the additives like aromatic essence, pigment, antioxidant or sequestering agent are added to the neat soap (melting soap with approximately 30 % of water) which was salted out after adding salt thereto and has become the soap material of 10 ∼ 15 % of water, having been through the drying step of pressure-reduction or hot air process, thus providing for even mixture of the materials to make the said soap.

Furthermore, when the liquid detergent is produced, the above said solid soap is to be added liquid components like glycerin to become liquid or the melting point may be lowered, shifting the types of oils and fats or by adjusting the volume of water the detergent may become liquid. The liquid detergent may as well be a foaming detergent comprising mainly ester alkylsulfate salt, betaine-type amphoteric surfactant.

The carbide of the hulls to be used for the present invention is a material rich in carbon (amorphous carbon) after being subject to the heat-dissolution wherein the hulls have been heated under adequate conditions. The carbide like this can be produced by roasting them by steam with the temperature of 300 ∼ 500 °C while shutting off the air.

In addition, the ash-like powders of the hulls to be used for the present invention are remnants of the solid ashes produced by roasting the hulls completely under good conditions.

Thus, ash-like powders of the hulls or carbide powders of the chaffs can be produced in good terms even by adjusting the volume of current air when roasting as below.

The dried hulls are to be roasted while they are heaped like a mountain on the earth outside. Then, the volume of the current air will be increased by raising the ventilating device in the shape of the chimney so that the said device protrudes toward the top of the mountain of hulls thus heaped from inside it. The ventilating device in the shape of the chimney may be provided with a bowl part comprising porous plates, wherein the chimney in the shape of an iron cylinder is connected on the top part of the said bowl part, thus providing for good results.

In order to produce the carbide powders of hulls by using the above-defined device, the source of fire like a dry lumber is first burned on the earth, above which, the bowl part of the ventilating device in the shape of a chimney with the above said structure is put on so that hulls are put over the said bowl part like a mountain to keep them burning.

In this way, the surface of hulls thus heaped like a mountain is getting gradually burned unevenly, therefore the hulls are stirred by the bars to make the whole part evenly burned black and when they are extinguished by throwing water, the carbide of hulls can be obtained.

In addition, at the above stage when the hulls are burned until they become ash-white without extinguishing, ash-like products of hulls can be made.

Then, the carbide of the hulls obtained or the ash-like products of hulls are to be pulverized by a milling step by means of an electric rotating blade for pulverization or friction so that they become micro particles. The size of the particles may be approximately between 1 and 50 *µ* m or preferably 1 and 30 *µ* m and there should be adopted the appropriate diameter of the particles in respect of the items to be cleaned, taking into consideration the cleaning efficiency. In order to prepare the micro powders with a micro diameter of the particles as defined above, a boring mill may preferably be adopted as a pulverizing machine.

The carbide powders of the hulls, thus obtained or ash-like products of the hulls or the compound based on them are to be dispersed after stirring and mixing evenly with the above said solid or liquid detergent or surfactants.

In order to stir and mix the hulls completely, the mixing machine may preferably be used and when the liquid detergent is to be produced little by little, the pulverized solid soap and the carbide powders of the hulls or the ash-like products of the hulls or the compound based on them are mixed and heated by strong fire inside the heat-proof container, melting the solid soap and stirring and mixing them evenly with the hand.

When the solid detergent is to be produced, the ash-like powders of hulls or carbide powders of hulls or compound powders based on them are added to the solid detergent in market and at this time the ratio is 3 to 85 volume percent. When the ratio is small as to be below this given range, oils and fats and other dirty components cannot be absorbed so enough as has been expected and this is not good. On the other hand, when the ratio is larger than the above given range, the solid soap cannot be formed and the solid soap thus obtained becomes so hard that it cannot be easily dissolved when washing with water, and this is not good either.

When the liquid detergent is to be produced, ash-like powders of hulls or carbide powders of hulls or the compound powders based on both of them are added to the liquid detergent in market and at this time the ratio is 3 to 50 volume percent. As the ratio is smaller than this given range, oils and fats and other dirty components cannot be absorbed so enough as has been expected and this is not good. On the other hand, when the ratio is larger than the above given range, a dispersed state of the above-mentioned powders in the liquid detergent becomes unstable and the powders and liquid will be separated even during a short-term conservation and this is not good.

The detergent of the present invention thus produced is capable of removing oil and fat attached onto the human skin or hairs or the surface of the root of the hair or waste matters of oils and fats and the mixture of other alien materials.

Out of the detergent like this, there can be produced a shampoo capable of growing the hairs in good health and a shampoo for getting rid of the odors of dogs and cats and other pet animals and there can be produced a detergent for washing, excellent in cleaning effects toward the collar parts where skin oils are hard to get rid of, especially.

### [Example 1]

Using the above said ventilating device in the shape of a chimney, the ash-like product was produced by heating hulls outside for three hours under good conditions. This product was pulverized by the electric rotating blade of the cooking mill and the powders obtained were added to the pulverized cosmetic soap (solid one) seen in market by 40 volume percent and these were stirred and mixed after being put into the frying pan as they were heated in the gas range for 20 minutes and then the melted liquid compound was put into the heat-proof container and got cooled while being left alone, thus providing for a solid detergent in the shape of a short column.

### [Example 2]

The solid detergent was obtained according to the example 1, except for the following conditions, wherein the hulls were heated for 2 hours outside under good conditions and when the whole part of hulls turns black, the carbide is produced by throwing water thereon and stop roasting them to pulverize them by a cooking mill provided with an electric rotating blade, using powders obtained.

### [Example 3]

The liquid detergent (cosmetic liquid soap) seen in market was added 30 volume percent of the ash-like powders of the hulls which have been produced in the same manner as in the example 1 and they were mixed by a cooking mixer and combined in a dispersed state to produce a liquid detergent.

### [Example 4]

The liquid detergent (cosmetic liquid soap) seen in market was added 30 volume percent of the carbide powders of the hulls which have been produced in the same manner as in the example 1 and they were mixed by a cooking mixer and combined in a dispersed state to produce a liquid detergent.

As for the detergent obtained as above according to the examples 1 to 4, five male and female samplers tried using it when washing faces and hands and they answered that oils disappeared definitely from the surface of the skin upon cleaning once, thus cleaning the sweat gland of the hair gland of the skin also, so that the skin respiration became possible, allowing for a fresh feeling.

### [Examples 5 and 6]

The liquid detergent was produced, precisely, according to the example 3, except for the conditions wherein water (example 5) or glycerin (example 6) was used instead of the liquid detergent seen in market.

### [Examples 7 and 8]

The liquid detergent was produced, precisely, according to the example 4, except for the conditions wherein water (example 7) or glycerin (example 8) was used instead of the liquid detergent seen in market.

When a windshield of vehicles was washed using the detergent in the examples 5 to 8 as above obtained, the whole part of the windshield was evenly washed with cleaning once and it was confirmed that the an oil film has disappeared. Also, according to the examples 7 and 8, the specified detergent was capable of using for washing the face and hands so that the oils disappeared definitely from the surface of the human skin with cleaning once, thus making the sweat gland also clean.

### [Effects of the Invention]

As described above, the detergent of the present invention has a merit that the dirty spots oil and fat attached onto the surface of the human skin can be absorbed evenly and removed definitely, as ash-like powders of hulls or carbide powders of hulls or the compound powders based on both of them are combined with the surfactants in a dispersed state.

The present invention concerning the above surfactants or solid detergent or glycerin or other liquid detergents, wherein the ash-like powders of hull or carbide powders of hulls are combined in a dispersed state, has a merit that it is capable of surely obtaining the above-mentioned cleaning effects without harming the soft surface of the materials to be cleaned other than the human skin.

In addition, the present invention concerning the detergent, wherein the given amount of the ash-like powders of hulls or carbide powders of hulls and others are combined in a dispersed state, adopting water instead of surfactants, has a merit of having even and definite cleaning effects and effects of removing oil films, thus providing for even absorption and removal of oil and fat.

## Claims

1. A composition comprising ash-like powders of hulls, carbonised or pyrolysed powders of hulls, or a combination of the two, dispersed in one or more surfactants.

2. A detergent composition according to claims 1 comprising a solid detergent in which are dispersed 3 to 85 volume percent, based on the total composition, of said hull powders.

3. A detergent composition according to claims 1 comprising a liquid detergent in which are dispersed 3 to 50 volume percent, based on the total composition, of said hull powders.

4. A detergent composition comprising ash-like powders of hulls, carbonised or pyrolysed powders of hulls, dispersed in water in an amount of 5 to 85 volume percent.

5. A detergent composition according to any preceding claim comprising powders of carbonised hulls producing by heating the hulls in steam at 300 to 500°C, with the exclusion of air.

6. A detergent composition according to any preceding claim comprising ash-like powders of hulls produced by roasting the hulls in air.

7. A detergent composition according to any preceding claim wherein said hulls are derived from the ears of rice plants.
